# EUROPEAN PATENT APPLICATION

(11) **EP 4 439 104 A1**
(43) Date of publication of application: **02.10.2024**
(21) Application number: 23164266.1
(22) Date of filing: 27.03.2023
(51) Int. Cl.: G01R 33/28, G01R 33/385, G01R 33/54

(54) **POWER CONTROLLER, SYSTEM AND METHOD FOR MEDICAL IMAGING SYSTEMS**

(71) Applicant: Koninklijke Philips N.V., 5656 AG Eindhoven (NL)
(72) Inventor: LEUSSLER, Christoph Günther, Eindhoven (NL); TERMEER, Martijn Krelis, Eindhoven (NL); WEISS, Steffen, 5656AG Eindhoven (NL); KOEHLER, Thomas, Eindhoven (NL); LIPS, Oliver, Eindhoven (NL); VOGTMEIER, Gereon, Eindhoven (NL)
(74) Representative: Philips Intellectual Property & Standards

(57) **Abstract**

Power control system (1), comprising a power controller (318) and a processor (324) for a medical imaging system (2), the medical imaging system (2) comprising a plurality of subunits (3) for acquiring and/or processing medical image data, a power supply unit (106) connected to the power control system (1), power distribution means (312) for providing electrical power from the power supply unit (106) to the subunits and data transmission means (322) between the power control system (1) and the subunits (3), wherein the power control system (1) is configured to receive information about the status and/or power consumption of a subunit (3), the power control system (1) is configured to adjust the power status and/or power consumption of a subunit (3) and the power control system (1) is configured to adjust and/or predict the status and/or power consumption of a subunit (3) with a self-optimizing and/or self-learning optimization algorithm (7) based on a usage history and/or environmental data. In this way, a power control system with improved characteristics is provided.

## Description

### FIELD OF THE INVENTION

The invention relates to the field of power distribution in a medical imaging system. In particular, the present invention relates to the field of power distribution systems, a switching device for sharing power units and further subunits and methods for medical imaging systems such as a magnetic resonance or X-ray imaging system.

### BACKGROUND OF THE INVENTION

Energy consumption in imaging systems is an increasing topic of attention. The impact on the environment and total cost of ownership (TCO) are becoming more and more important. Large scale management of imaging equipment fleet can contribute to optimization of TCO in a hospital or hospital chains. In addition, in general certain critical system components require a backup component or replacement to keep the entire system running in case of failures of individual power units or amplifier units or high voltage X-ray generators which usually comprise a high voltage power supply for an X-ray source.

Typically, magnet resonance tomography (usually referred to magnet resonance: MR) or computed tomography systems are managed in a passive way. The system is powered up when the user logs in, and the system is set to standby after a fixed time after a last scan. This is not optimal in terms of workflow, the availability of the system and in terms of energy consumption.

Using artificial intelligence (AI) with deep learning algorithms and optional sensors it is generally possible to optimize the energy management and availability of systems in an automated way and adapt it to the user. AI allows that a system learns the working habits of the operators and makes choices that reduce the energy usage.

There is prior art on monitoring energy consumption based on logfile analysis and power consumption models of the MR clients. This gives insights in the usage of the magnetic resonance imaging (MRI, or shorter MR) systems and MRI fleet, and enables optimization in management and development, but it is retrospective of nature.

Gradient Amplifiers provide power to generate the variable (gradient) magnetic field component for the MR imaging process. The gradient amplifiers in magnetic resonance imaging (MRI) drive the gradient coils with currents of several hundred amperes to create the so-called gradient fields used in the imaging process. These are magnetic fields generated by the gradient coils which are designed to create fields that have a spatial gradient in a certain direction (x,y, or z). Therefore, each MRI system has three gradient coil channels for x, y, and z direction each equipped with a gradient coil connected to a gradient amplifier.

Often several MRI or computed tomography systems - which may also include X-ray scanners - are operated in one hospital. For instance, each MRI scanner is equipped with a full set of gradient amplifier hardware, and the gradient amplifier is a major item in the bill of materials of the system. If one of these gradient amplifiers fails, the MRI scanner is immediately out of service. In X-ray imaging systems this applies for instance to high voltage X-ray generators which include a high voltage power supply for an X-ray source.

During an MRI scan very high gradient power is only required for parts of the image acquisition sequences. MR scan preparation, survey, etc. and many other clinical routine investigations require only moderate gradient power, whereas diffusion scans and high-resolution scans (cardiac MRI) etc. need high gradient power (e.g. 80 mT/m).

Modern shielded MRI systems can be located relatively close to each other with a center-to-center distance of about 4 m. Trends are also to easily replace components when newer, better devices are available - also there the switching matrix with calibration intelligence could stay the same and amplifiers can be replaced.

Most scans require a high switching speed of the gradients which means that the amplifier must deliver a high voltage. Some scans however require the gradient coils to be driven with very high currents to achieve high gradient field strength (diffusion scans). Some MRI system are therefore equipped with two gradient amplifiers per channel (e.g. x-channel), and these can be connected to the (x-gradient) coil either in series to provide high voltage or in parallel to provide high current. Respective switching is performed by SW-controlled semiconductor-based current switches. Typically fixed installed leads and non-pluggable connectors are used for managing high currents as several 100 A, which is also the case in standard MRI gradient systems.

WO2010052616 describes a MRI system comprising a power supply unit adapted for providing direct current electrical power and a power controller that is connected to a computer allowing an automated power distribution and a configuration by a user via a user interface.

### SUMMARY OF THE INVENTION

It is an object of the invention to provide a smart distribution of power to a medical imaging device which allows an improved workflow or availability of the system and advanced energy saving capabilities.

According to the invention, this object is addressed by the subject matter of the independent claims. Preferred embodiments of the invention are described in the sub claims.

The invention relates to a power control system comprising a power controller and a processor for a medical imaging system, the medical imaging system comprising a plurality of subunits for acquiring and/or processing medical image data, a power supply unit connected to the power control system, power distribution means for providing electrical power from the power supply unit of the power control system to the subunits, data transmission means between the power control system and the subunits, wherein the power control system is configured to receive information about the status and/or power consumption of a subunit, and the power control system is configured to adjust the power status and/or power consumption of a subunit preferably via the power controller, wherein the power control system is configured to automatically adjust and/or predict the status and/or power consumption of a subunit with a self-optimizing and/or self-learning optimization algorithm based on a usage history and/or environmental data.

The optimization algorithms may have self-learning intelligence using machine learning and artificial intelligence technology, enabling to attain and preferably maintain optimal, close to optimal, or otherwise enhanced performance. A self-optimizing or self-learning optimization algorithm comprises machine learning (ML) which is a field of inquiry devoted to understanding and building methods that learn. This learning process is seen as a part of artificial intelligence and typically include methods that leverage data to improve performance on some set of tasks. Machine learning algorithms usually have or build a model based on sample data, known as training data, in order to make predictions or decisions without being explicitly programmed to do so.

A self-optimizing optimization algorithm usually comprises an algorithm which can adjust a parameter or a set of parameters, used for calculation of a predetermined target value, in such a way, that the target value is minimized or maximized. Hence this may include an automatic adjustment of the control parameters and/or input variables which can originate for instance from the environment and/or sensors, predetermined start values and/or a user. Therefore, a self-optimizing optimization algorithm can comprise a process in which the system's settings can be autonomously and continuously adapted to ongoing processes and to changes in the environment even after an initial optimization which may have been performed at the beginning. The notion self-learning optimization algorithm puts emphasis on learning capabilities.

The aforementioned concept is not restricted to power distribution and switching of power to certain medical imaging systems at a medical imaging site, but the concept may also be applied to the sharing or switching of subunits to a required medical imaging systems which is, for instance, currently in use. Hence the general concept may also relate to the sharing or switching of functional modules or functionalities of subunits.

Preferably the power control system is configured to receive data from a subunit and/or a sensor unit. This can be realized by additional wired or wireless connections which may comprise a network. Also, existing connections or powerlines may be used to transfer information by adding signals to an existing connection. Added connections typically require additional receiver and transmitter modules. By receiving information from a subunit and/or a sensor unit, the power control system can monitor the units and obtain a status of the system. This allows the power control system to react to changing conditions, but also to learn and perform artificial intelligence or machine learning algorithms including self-optimizing and/or self-learning optimization algorithms.

According to a preferred embodiment of the invention the power control system is configured to receive data from a user interface and/or a host. A user interface allows the user to configure and control the power control system. For automatic routines and/or configurations including updates, but also for extended control possibilities a connection to the host is of advantage. The connection is typically bidirectional connection but can also be unidirectional. The data can comprise all types of information, for instance commands, but also system settings, environmental data and sensor data etc.

Preferably the power control system is configured to receive data comprising information about the pressure of the coolant inside the magnet /helium vessel and/or the required gradient field strength and/or availability of mains power and/or a cooling function. This allows the power control system to use and consider the highly significant data about one of the most central components given by the pressure of the coolant, the availability of mains power and the cooling function.

Preferably the power control system is configured to reduce the power consumption and/or a workflow duration of the medical imaging system. It is preferred that the power control system, especially the optimization algorithm, has such an optimization function which targets to minimize the power consumption and/or the workflow duration of the medical imaging system. The power consumption may be competing with the workflow duration. Depending on the optimization function of the optimization algorithm, usually a trade-off between both parameters needs to be set.

According to another preferred embodiment of the invention the power control system is configured to reduce a standby time and/or a start-up duration of medical imaging system. Also, these two target parameters may significantly influence an automatic control of the medical imaging system and usually compete with each other. It is also possible that the main target parameter is the power consumption, and the algorithm is considering further target parameters with a different or lower weight, for instance the afore-mentioned parameters like the standby time, workflow duration and start-up duration.

Preferably, the power control system comprises a deep learning algorithm which is trained on neural networks. While deep learning algorithms in principle feature self-learning representations, they often depend upon artificial neural networks, which mimic the way the brain computes information. During a typical training process, the related algorithms use unknown elements in the input distribution to extract features, group objects, and discover useful data patterns. Similar to training machines for self-learning, this occurs at multiple levels, using the algorithms to build the models. Deep learning models usually make use of several algorithms. For most application scenarios none of the most used algorithm types is considered universal, hence a combination is often chosen. Types of typical algorithms used in deep learning are convolutional neural networks (CNNs), long short term memory networks (LSTMs), recurrent neural Networks (RNNs), Generative Adversarial Networks (GANs), Radial Basis Function Networks (RBFNs), Multilayer Perceptrons (MLPs), Self-Organizing Maps (SOMs), Deep Belief Networks (DBNs), Restricted Boltzmann Machines (RBMs) and Autoencoders. Deep learning algorithms typically work with any kind of data and require much information to solve multi parameter tasks.

The invention further comprises a magnetic resonance imaging (MRI) system or arrangement, wherein an MRI arrangement may comprise more than one MRI system, with a magnetic resonance (MR) scanner, a gradient amplifier, a processor, a memory for storing machine executable instructions for execution by the processor, pulse sequence commands and the power control system. MR imaging systems typically have a magnetic resonance scanner which usually require a gradient amplifier for generating the strong gradient magnetic field. Different types of MR scans with different image contrasts are performed by playing out pulse sequence commands. Some of these commands define the complete temporal waveform of the gradient field strength generated by the gradient coils.

Preferably a magnetic resonance imaging (MRI) site or arrangement, referring to a fleet of one or more MR imaging systems in a radiology department, comprises a plurality of magnetic resonance systems and hence a plurality of MRI scanners and a plurality of gradient amplifiers, wherein the power control system is configured to switch the gradient amplifiers flexibly to any magnetic resonance scanner as required in a respective use case. This flexibility allows for switching the gradient amplifiers to any magnetic resonance scanner or not, depending on the respective use case. This allows to share gradient amplifiers with several MRI scanners or MRI systems on a case-by-case basis. For instance, a switch matrix or an active selection switch may distribute the gradient performance, mainly given by the amplitude, from the gradient amplifier modules to the MR scanners of a MRI site in use. An interface may allow to plug additional, possibly mobile gradient amplifiers. Such a system allows modular energy distribution on - preferably parallel - managed MR scanners of a MRI site with more than one MRI system. Further an active switch board matrix may distribute the gradient power from the gradient amplifier modules to the MR scanners of a respective MRI system in use.

According to another preferred embodiment of the invention the plurality of gradient amplifiers of the MR imaging system or arrangement comprises a number of *n* gradient amplifiers which are configured to be shared by a number of *m* magnetic resonance scanners of the plurality of magnetic resonance scanners, with *n* less or equal than 3^{∗}*m*. Mainly due to technical requirements usually a number of three gradient amplifiers is used for one magnetic resonance scanner.

In a further embodiment of the invention the power control system of the magnetic resonance imaging system or arrangement is configured to switch and/or connect any subunit of the magnetic resonance imaging system flexibly to any magnetic resonance scanner as required. Hence the power control system can directly or indirectly distribute the functionality of a subunit to a specific scanner or part of an MRI site, for instance upon functional request. In the general, the same applies to X-ray imaging systems and subunits of radiology departments with several X-ray imaging systems. The subunits preferably comprise not only the gradient amplifiers, but also high voltage generators in case of computed tomography systems and any further needed supply or assisting module that is - preferably temporarily - required for the operation of a medical imaging device.

Preferably the power control system of the magnetic resonance imaging system or arrangement is configured to distribute scans in time such that the use of the gradient amplifiers is distributed evenly over time. In this way, power consumption peaks as well as the number of gradient amplifiers - in case of a plurality of scanners and shared amplifiers - may be reduced. Hence the overall effort and costs can be significantly reduced.

According to a preferred embodiment of the invention the gradient amplifiers of the magnetic resonance imaging system or arrangement comprise high voltage and/or high current amplifiers. These two types of amplifiers are often used in magnetic resonance imaging systems.

The invention further comprises a method for operating a power control system with a power controller and a processor for a medical imaging system or arrangement comprising a plurality of subunits for acquiring and/or processing medical image data, wherein the power control system receives information about the status and/or power consumption of a subunit, and the power control system - preferably automatically - adjusts and/or predicts the status and/or power consumption of a subunit with a self-optimizing and/or self-learning optimization algorithm based on a usage history and/or environmental data.

According to a preferred embodiment of the invention the method for operating a power control system comprises a plurality of magnetic resonance imaging scanners. In preferred embodiments the method for operating a power control system may perform or comprise any of the characteristics, steps and actions which have been described related to the power control system, the medical imaging system and the magnetic resonance imaging system.

### BRIEF DESCRIPTION OF THE DRAWINGS

These and other aspects of the invention will be apparent from and elucidated with reference to the embodiments described hereinafter. Such an embodiment does not necessarily represent the full scope of the invention, however, and reference is made therefore to the claims and herein for interpreting the scope of the invention.

In the drawings:
Fig. 1 schematically depicts a power control system of a medical imaging system according to a preferred embodiment of the invention,
Fig. 2 schematically depicts an example for AI controlled resource management of a fleet of several MRI scanners,
Fig. 3 schematically depicts a medical imaging system or arrangement with a power control system and several subunits according to a preferred embodiment of the invention,
Fig. 4 schematically depicts a medical imaging system or arrangement according to a preferred embodiment of the invention, with three MRI scanners sharing the same modular gradient amplifier system,
Fig. 5 schematically depicts an exemplary switching flow algorithm of a preferred embodiment of the invention,
Fig. 6 schematically depicts an example for optimized scheduling for gradient hardware (HW) of a preferred embodiment of the invention and
Fig. 7 schematically depicts the state machine to control the process of simultaneous configurable gradient power of a preferred embodiment of the invention.

### DETAILED DESCRIPTION OF EMBODIMENTS

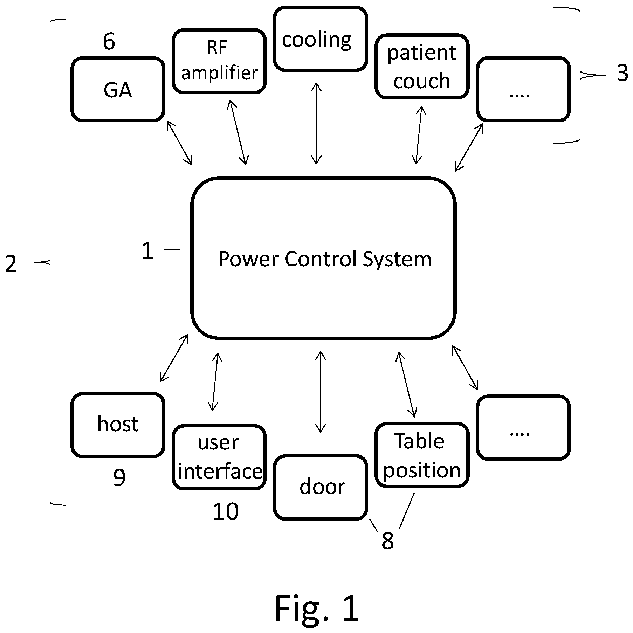

Fig. 1 schematically depicts a power control system 1 of a medical imaging system 2 according to a preferred embodiment of the invention. The power control system 1 delivers power to the connected modules and exchanges information with the connected modules of the medical imaging system 2, for instance with the subunits 3 comprising a gradient amplifier 6 (GA), a radio frequency (RF) amplifier, the cooling system and the patient couch. Further information is exchanged between the power control system 1 and the host 9, the user interface 10 and sensor units 8 like for the door and/or table position.

Current imaging devices already consist of many submodules. The use of modular architectures is additionally becoming increasingly popular. It is proposed that each of these submodules becomes "smart" and can be switched into a standby or a low power mode (e.g. where capacitor banks remain charged, but the rest is in standby). Moreover, it should be able to report its status and at least its current energy consumption data (e.g. currents and voltages). Especially for MRI further, more specific typical examples of such a module - additional to those mentioned already above - could be the RX chain, the cryocooler, displays, reconstruction computer. The communication can be realized by standard network protocols.

The core of the proposed embodiment is a controller, that employs an artificial intelligence (AI) model to predict when the different system modules will be needed. Furthermore, it analyzes or estimates how long the system modules need to power up, so a very efficient adoption to the required needs can be realized. A schematic overview is provided in Fig. 1.

For its prediction model and/or the required training phase of the AI-based methods the controller utilizes as features input from various sources, "classical" sensor data like the table position sensor, the door sensor, connected coils, etc. and possibly more important data from the host computer including the user interface. It can be analyzed, what scans are planned, in which state the planning is, what coils will be required etc.

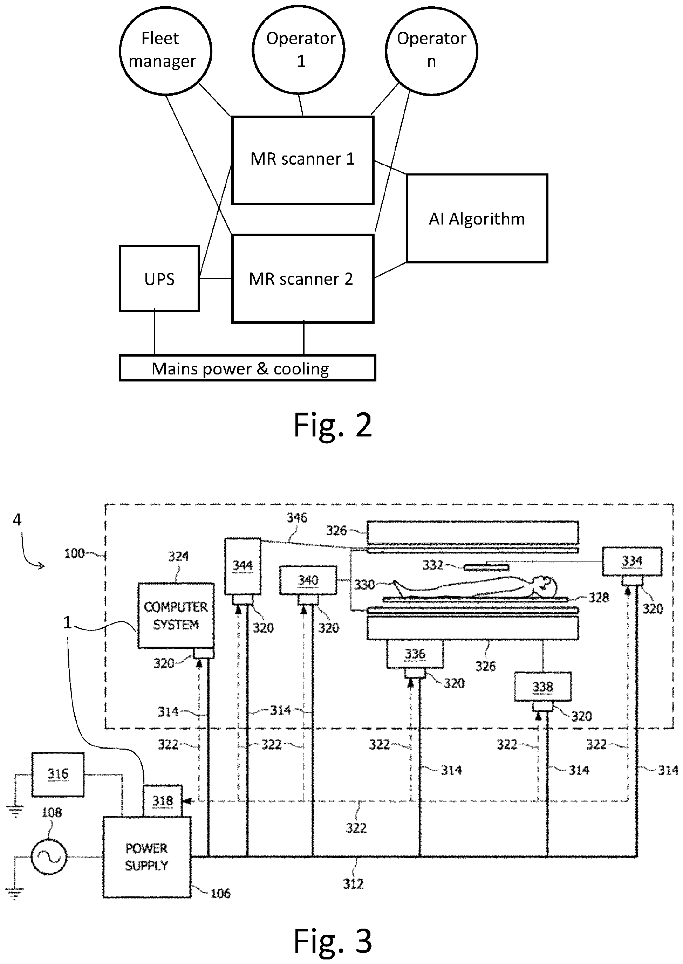

Fig. 2 schematically shows an exemplarily control concept. The AI algorithm is running on one or multiple MR systems, or on a separate computer platform. The MR scanners are connected to mains power and external cooling flow. Optionally one or more MR scanners are completely or partly backed up against power outage by one or more uninterruptible power supplies (UPS's). The operators and fleet managers interact with the scanners.

The AI algorithm can monitor the energy consumption and use it to learn and act. It knows this either by using sensor information of the taken up power from mains and optional UPS, or by log-files and power models. Also a combination of model and measurement is possible. It is also possible to give each subunit (such as gradient and RF amplifier) a power monitor. This feeds more detail to the AI algorithm.

The AI algorithm knows the planning of the system and can already get the system up and running for the first patient on the day. This reduces setup time with minimum energy consumption increase. It can also set the system into standby, for example during lunch pause, depending on state of the MR scanner and operator behavior.

Like with other computers, the choices of the AI algorithm can be made manually over-rideable, so that it is always possible to manually bring the system in operational state or put it back to standby. The AI helps the user to make energy consumption reduction more easily, in an automated way. Feedback on energy reduction can be given to the customer, for example in the form of a dashboard. Suggestions to further reduce energy usage can be given to the operator of the MR scanner, or to the fleet management of the hospital.

There is a difference in subunits of the MR system in how their energy usage depends on the usage of the scanner. The cryo-compressor needed to cool the cryogenic superconducting magnet, is typically only slightly depending on the scanner activity. It always has a relative high energy consumption, while the rest of the clients such as gradient and RF amplifiers can be in a low energy mode when there is no scanning activity. The AI algorithm learns these differences automatically, since it knows the energy consumption and knows the output of the scanner in terms of patient throughput. For a good performance of such AI algorithms already at startup or delivery to a customer, an AI algorithm is usually pre-trained.

Optimization of power management in a Smart Grid approach can be realized. The algorithm knows day/night patterns, and can for example be coupled to information on solar panels/green energy source availability. Also it can decide to charge UPS's during the day, when the system is in standby or running low demanding scans.

Automated and optimized decisions can be taken on power outage, for example the management of a UPS of one or multiple systems, can be taken over by the AI algorithm. Different choices can be made, depending on whether the mains power and external coolant flow is down (flow and/or pressure sensor information is needed), or if only the mains power is down.

This invention focuses on MR scanners, but the same approach can be used for other imaging modalities. The AI switching method could be detected by observing the behavior of the system or scanner, by code review or by commercial claims or training material. Extra HW sensors (such as temperature sensors, flow sensors, power monitor/sensors) could be detected, but it is difficult to judge whether these feed a conventional algorithm or an AI algorithm.

Fig. 3 shows a more detailed schematic of an embodiment of a medical imaging system 2, in particular a MRI system 4, in the following also referred to as MRI arrangement 4, with a related power control system 1. In this embodiment, there is an alternating-current (AC) electrical mains 108 which is connected to the power supply 106. Also connected to the power supply 106 is an UPS 316. The UPS 316 comprises one or more batteries and is adapted for providing DC electrical power to the power supply 106 and the power bus 312 in the event that AC power is lost. The power supply 106 is connected to the power bus 312 and it is also connected to the power supply controller 318. The power supply controller functions as part of the control means for controlling the supply of DC current to the power bus 312. The power supply controller 318 is connected to a communication network 322. This can be implemented using a computer network such as an ethernet. The power bus 312 itself can also be used to transport data. This can be implemented by incorporating network cable into the power bus or by imposing a high frequency carrier onto one or more of the conductors used for transmitting electrical power. The high frequency carrier is a low amplitude voltage superimposed upon the conductor that is adapted for transmission of data, but does not affect the transmission of power to the subunits 324, 334, 336, 338, 340, 344. In this embodiment the network 322 is connected to the data acquisition means 100 and also the power bus 312 is connected to the data acquisition means 100. There is a connection between the power bus 312 and a power bus controller 320. There is a network connection 322 between the power supply controller 318 and the computer system 324 as well as the power bus controller 320. In this embodiment the computer system 324 has a direct connection with the power supply controller 318.

In this embodiment the computer system 324 can run algorithms that perform automatic self-optimization and/or self-learning algorithms based on the history of data related to the user and/or sensor and/or subunit. The computer system 324 can further interact with the power supply controller 318 and forms part of the control system for regulating the power in the MRI system. This could also be implemented with power supply controller 318 controlling the power bus controllers 320 directly. The data acquisition means 100 has a computer system 324, a liquid cooling cabinet 344, a gradient power supply 340, a cryocooler 336, a magnet power supply 338, and an RF amplifier and/or receiver 334. Each of these subunits 324, 334, 336, 338, 340, 344 is connected to a power bus controller 320. Each power bus controller 320 is connected to an electrical connection 314 between the power bus 312 and the subunit 324, 334, 336, 338, 340, 344 and has a network connection 322. All of these subunits 324, 334, 336, 338, 340, 344 are connected to the power bus 312 and receive DC electrical power and are connected together through a computer network 322. The computer network 322 in this embodiment is operable for regulating the DC power each of the subunits 324, 334, 336, 338, 340, 344 receives.

The DC power can be shut off to individual subunits 324, 334, 336, 338, 340, 344 or a signal can be sent to the power bus controller 320 over the network 322 to regulate the power. The DC power bus 312 can supply a single electrical voltage and each of the units receives the same DC voltage. In this case some units can have DC to DC converters for producing different voltages. In an alternative embodiment, the power bus 312 can provide several different voltages.

In Fig. 3, there is also an MRI magnet 326. Within the MRI magnet 326 there is a patient support 328 for receiving a patient 330. The patient support 328 supports the patient during an examination or treatment. There is an RF transceiver coil 332 above the patient 330 and is connected to the RF amplifier 334. Inside the bore of the magnet, there are gradient coils 342. The gradient coils 342 are connected to the gradient power supply 340. There is a system of cooling hoses 346 connected to the gradient coils 342 These cooling hoses 346 connect the liquid cooling cabinet 344, which is adapted for cooling the fluid which is inside of the cooling hoses 346, and the gradient coils 342. The MRI magnet 326 is also connected to the magnet power supply 338. The magnet power supply 338 is capable of powering or depowering the MRI magnet 326. The magnet power supply 338 can be left in place at all times, or it can be removable. The magnet power supply 338 can be operated also without the network connection 322 and without the power bus controller 320. This would be the case if the magnet power supply 338 is being used as a service tool. A cryocooler 336 is used to cool the MRI magnet 326, and it is connected to the MRI magnet 326 using a thermal conduit.

In another embodiment, the subunits 324, 334, 336, 338, 340, 344 and the power supply controller 318 are not all on the same computer network 322. There can be dedicated lines running between the computer system 324 and the power supply controller 318 and to the different subunits 324, 334, 336, 338, 340, 344. For example, there may be a connection for each of the individual subunits 324, 334, 336, 338, 340, 344 and there may also be dedicated lines running from the power supply controller 318 to the power bus controllers 320.

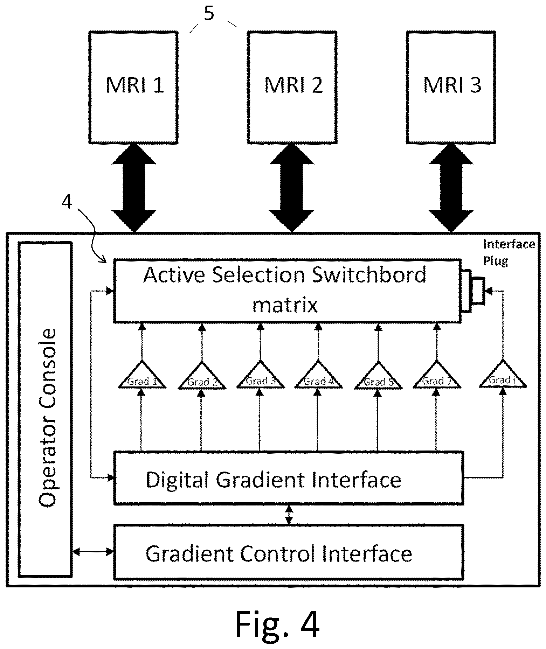

Fig. 4 schematically depicts a medical imaging system according to a preferred embodiment of the invention, with three MR scanners sharing the same modular gradient amplifier system. An active selection switch distributes the gradient performance, which usually means the amplitude, from the gradient amplifier modules to the MR scanners of a MRI site in use. An interface allows to plug additional mobile gradient amplifiers.
Further Fig. 4 illustrates an MRI site with multiple MRI scanners 5 possibly of different field strength, which share a pool of dedicated gradient systems for diagnostic and therapy imaging. The system allows modular energy distribution on parallel managed MR scanners.

As shown in Fig. 4 three MR scanners share the same modular set of gradient amplifiers. An active switch board matrix distributes the gradient power from the gradient amplifier modules to the MR scanners in use. In case of demand of high gradient power or high voltage, corresponding amplifiers or submodules are selected and switched in series or multiple amplifier modules are operated in parallel.

The active selection switch can be configured as follows: 1.) Aa a stand-alone unit, which is coupled with stand-alone gradient amplifiers 6 and the MR scanner 5. 2.) Integrated in a rack with different gradient modules or amplifiers 6. The rack supplies different MR scanners. 3.) As a switch board matrix which is distributed, such that part of the active switch is located at the individual MR scanner 5.

MR scanners 5 can be run simultaneously. Direct exchange of information and synchronization between subcomponents is performed by a switching algorithm which runs on the gradient control interface. The interface administrates system information, control parameters, specifications of amplifiers characteristics, details of switch matrix and gradient coil limits. Requests of MR console SW interface are controlled and streamlined to provide highest safe and valuable diagnostic and clinical service. The different input waveforms with respect to the simultaneous MR sequences (here MR sequence 1-3) are distributed by the digital gradient control interface.
The proposed MR control software interface is active at *m* different user consoles (e.g. one per scanner) or emulated remote consoles. This allows the users to run the MR sequences on any of the MR scanners 5 connected to the gradient switch matrix. The MR scanners 5 can be run simultaneously.

Different gradient amplifiers 6 may share the same power supply, stored energy, or power supply generator.

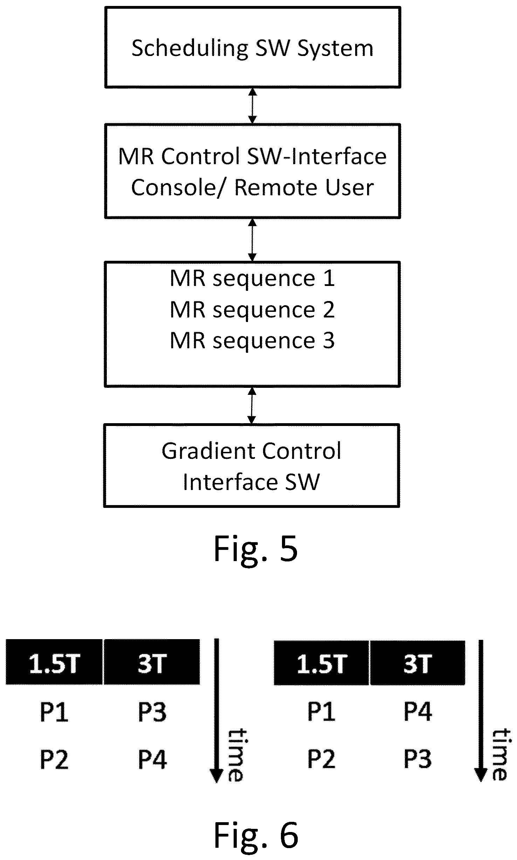

An AI-based algorithm to infer scheduling based on sensor data (gradient switch) and context information (service, status, function, authorization) and to predict the development of this state. A display apparatus or software window to inform the operator about the current and predicted state of the system. A decision logic is additionally implemented to automatically stop the MR scan when emergency situations are detected or predicted by the sensor data. Fig. 5 shows a schematic of an exemplary switching flow algorithm.

In another embodiment as shown in Fig. 6 a system for scheduling of exams and scans are adapted to share existing gradient hardware (HW) and to make time-optimized use of part-time rented mobile hardware.

This embodiment offers advantages related to the fact that only few scans (e.g., diffusion scans) require full gradient power and is enabled by novel plug & play gradient amplifiers allowing exchange between MR scanners in seconds.

An example for optimized scheduling for existing gradient hardware is given by the following configuration: Two scanners (1.5T, 3T) are side by side; there are 4 patients (P1 to P4) to be scheduled, patients P1 and P2 are on 1.5T and patients P3 and P4 are on 3T; patients P1 and P3 require a diffusion scan which requires high power gradients. A standard approach would be to schedule patients straight forward meaning that each scanner must be equipped with a high-power amplifier. According to the invention, the system rather calculates a new schedule under the constraint to not use high power amplifiers at both systems simultaneously and hence the systems can now share one low and one high power amplifier.

An example for a time-optimized use of part-time rented mobile modular HW can be as follows:
This concept can be based on rentable mobile plug & play gradient amplifiers on wheels. The system checks the upcoming demand for high power scans and the availability of rentable amplifiers 6. Then the system schedules patients with diffusion scans in a respective time window of a month, for example.

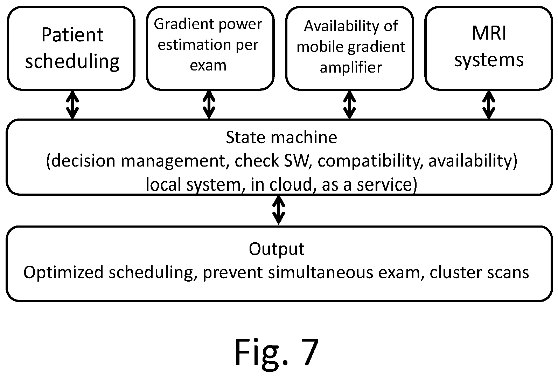

Fig. 7 depicts a schematic of a state machine for decision management which controls different settings with respect to access, locking, blocking. Input parameters are interactive patient scheduling, gradient power estimation, individual gradient power amplifier status and of the MRI system.

The gradient rack and/or the gradient switch matrix is equipped with a connection interface to allow the connection of a mobile amplifier (see also Fig. 4). Mobile amplifiers 6 allow to upgrade the system performance or to allow continuation of clinical service in case of individual amplifier shutdown to prevent system interruption. The amplifiers 6 are connected by a smart plug interface, which is controlled by the gradient control interface. Compatibility management and request is handled by state machine and scheduling software module. A SW management tool controls the correct interfacing of the dedicated gradient device (guidance to connect, calibration, store system settings, system performance).

In standard MR scanners with fixed gradient systems the gradient systems are calibrated on installation. This means that for each channel the gradient amplifier current output is fine-tuned by slight adjustments of the demand waveform from its nominal value to exactly deliver the required current waveform to its permanently connected gradient coil. This calibration is stored and then used for all subsequent scans. The calibration needs update only if the amplifier or the coil is exchanged.
For a shared pool of amplifiers, it is proposed to perform this calibration for all possible (or in practice used) combinations of amplifiers and coils. All calibrations are stored and applied if the respective combination is realized per scheduling software. The software prevents connections, which are uncalibrated.

Replacement of same and better amplifiers or better devices is identified by an intelligent calibration manager and calibration is updated.
In a special embodiment it is also possible to predict calibrations based on some simple reference measurements of the amplifier in combination with known calibration results for the specific gradient coil.

The concept of the invention can be applied for various imaging systems (i.a. MRI systems, X-ray imaging systems, microscopy systems etc.) and it is not restricted to them. There are existing different modalities where this concept of module sharing based on the estimated or scheduled usage and the selected protocols can also be applied including the already mentioned back-up functionality to keep imaging systems running across the cluster of systems.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope. Further, for the sake of clearness, not all elements in the drawings may have been supplied with reference signs.

**REFERENCE SYMBOL LIST**

| | |
|---|---|
| Power control system | 1 |
| Medical imaging system | 2 |
| Subunit | 3 |
| Magnetic resonance imaging system | 4 |
| Magnet resonance (MR) scanner | 5 |
| Gradient amplifier | 6 |
| Optimization algorithm | 7 |
| Sensor unit | 8 |
| Host | 9 |
| User interface | 10 |
| Power controller | 318 |
| Processor | 324 |

## Claims

1. Power control system (1), comprising a power controller (318) and a processor (324) for a medical imaging system (2),
the medical imaging system (2) comprising
a plurality of subunits (3) for acquiring and/or processing medical image data,
a power supply unit (106) connected to the power control system (1),
power distribution means (312) for providing electrical power from the power supply unit (106) to the subunits and
data transmission means (322) between the power control system (1) and the subunits (3), wherein
the power control system (1) is configured to receive information about the status and/or power consumption of a subunit (3),
the power control system (1) is configured to adjust the power status and/or power consumption of a subunit (3) and
the power control system (1) is configured to adjust and/or predict the status and/or power consumption of a subunit (3) with a self-optimizing and/or self-learning optimization algorithm (7) based on a usage history and/or environmental data.

2. Power control system of claim 1, wherein the power control system (1) is configured to receive data from a subunit (3) and/or a sensor unit (8).

3. Power control system of claim 1 or 2, wherein the power control system (1) is configured to receive data from a user interface (10) and/or a host (9).

4. Power control system of claim 1 to 3, wherein the power control system (1) is configured to receive data comprising information about the required gradient field strength and/or coolant pressure and/or availability of mains power and/or a cooling function.

5. Power control system of claim 1 to 4, wherein the power control system (1) is configured to reduce the power consumption and/or a workflow duration of the medical imaging system (2) and/or a standby time and/or a start-up duration of medical imaging system (2).

6. Power control system of claim 1 to 5, wherein the algorithm (7) comprises a deep learning algorithm which is trained on neural networks.

7. Magnetic resonance imaging arrangement (4) comprising a magnetic resonance scanner (5), a gradient amplifier (6), a processor, a memory for storing machine executable instructions for execution by the processor, pulse sequence commands and a power control system (1) according to any of the preceding claims.

8. Magnetic resonance imaging arrangement (4) of claim 7, comprising a plurality of magnetic resonance scanners (5) and a plurality of gradient amplifiers (6), wherein the power control system (1) is configured to switch the gradient amplifiers (6) flexibly to any magnetic resonance scanner (5) as required.

9. Magnetic resonance imaging arrangement (4) of claim 8, wherein the plurality of gradient amplifiers (6) comprises a number of *n* gradient amplifiers (6) which are configured to be shared by a number of *m* magnetic resonance scanners (5) of the plurality of magnetic resonance scanners (5), with *n* less or equal than 3^{∗}*m* gradient amplifiers (6).

10. Magnetic resonance imaging arrangement (4) of claim 8 or 9, wherein the power control system (1) is configured to switch any subunit (3) to any magnetic resonance scanner (5).

11. Magnetic resonance imaging arrangement (4) of claim 8 to 10, wherein the power control system (1) is configured to distribute scans in time such that the use of the gradient amplifiers (6) is distributed evenly over time.

12. Magnetic resonance imaging arrangement (4) of claim 8 to 11, wherein the gradient amplifiers (6) comprise high voltage and/or high current amplifiers.

13. X-ray imaging arrangement comprising a high voltage X-ray generator, a power unit for the high voltage X-ray generator and a power control system (1) according to any of claims 1 to 6.

14. Method for operating a power control system (1) with a power controller (318) and a processor (324) for a medical imaging system (2) comprising a plurality of subunits (3) for acquiring and/or processing medical image data, wherein
the power control system (1) receives information about the status and/or power consumption of a subunit (3), and
the power control system (1) automatically adjusts and/or predicts the status and/or power consumption of a subunit (3) with a self-optimizing and/or self-learning optimization algorithm (7) based on a usage history and/or environmental data.

15. Method of claim 14, wherein the power control system (1) controls a plurality of magnetic resonance imaging scanners (5).
